# EUROPEAN PATENT APPLICATION

(11) **EP 3 915 458 A1**
(43) Date of publication of application: **01.12.2021**
(21) Application number: 21175779.4
(22) Date of filing: 25.05.2021
(51) Int. Cl.: A61B 1/00, A61B 1/12

(54) **ENDOSCOPE INCLUDING A CLEANING ASSEMBLY**

(30) Priority: 26.05.2020 US 202016883455
(71) Applicant: Covidien LP, Mansfield, MA 02048 (US)
(72) Inventor: HOLSTEN, Henry E., Hamden, CT Connecticut 06518 (US); RICHARD, Paul D., Shelton, CT Connecticut 06484 (US); EBERSOLE, Garrett P., Hamden, CT Connecticut 06517 (US); BUDHABHATTI, Sachin P., Unionville, CT Connecticut 06085 (US)
(74) Representative: Maschio, Antonio

(57) **Abstract**

An endoscope includes a cleaning assembly that enables cleaning of a lens of the endoscope during a surgical procedure to maintain a clear image without having to remove the endoscope from the patient's body. The cleaning assembly includes an ultrasonic transducer operatively coupled to the lens to provide vibration thereto to remove fluids and debris from the lens, and an ultrasonic generator providing driving signals to the ultrasonic transducer.

## Description

### TECHNICAL FIELD

The present disclosure relates to a minimally invasive viewing instrument and, more particularly, to an endoscope including a cleaning assembly configured to remove fluids and debris from a lens of the endoscope.

### BACKGROUND

Minimally invasive surgery eliminates the need to cut a large incision in a patient, thereby reducing discomfort, recovery time, and many of the deleterious side effects associated with traditional open surgery. Minimally invasive viewing instruments such as, e.g., laparoscopes and endoscopes, provide viewing of internal tissues and/or organs during the minimally invasive surgery.

Laparoscopic surgery involves the placement of a laparoscope in a small incision in the abdominal wall of a patient, to view the surgical site. Endoscopic surgery involves the placement of an endoscope in a naturally occurring orifice, e.g., mouth, nose, anus, urethra, or vagina, to view the surgical site. Other minimally invasive surgical procedures include video assisted thoracic surgery and cardiovascular surgery conducted through small incisions between the ribs. These procedures also utilize scopes to view the surgical site.

A typical minimally invasive viewing instrument, e.g., a laparoscope or an endoscope, includes a housing, an elongated lens shaft extending from one end of the housing, and a lens that is provided in a distal end of the elongated lens shaft. A camera viewfinder extends from the other end of the housing. A camera is connected to the housing and transmits images sighted through the lens to an external monitor on which the images are displayed. During a surgical procedure, the distal end portion of the elongated lens shaft is extended into the patient, while the proximal end portion of the elongated lens shaft, the housing and the camera viewfinder remain outside the patient. In this manner, the laparoscope/endoscope is positioned and adjusted to view particular anatomical structures in the surgical field on the monitor.

During insertion of an endoscope or a laparoscope into the body and during the surgical procedure, debris, e.g., organic matter and/or moisture, may be deposited on the lens of the scope. The buildup of debris and condensation on the lens impairs visualization of the surgical site, and often necessitates cleaning of the lens.

### SUMMARY

The present disclosure describes an endoscope that demonstrates a practical approach to meeting the performance requirements and overcoming usability challenges associated with endoscopic surgery.

In accordance with this disclosure, an endoscope includes a housing coupled to a power source, an elongate tubular shaft extending from the housing, and a cleaning assembly. The elongate tubular shaft includes a distal end portion having a lens. The cleaning assembly includes an ultrasonic transducer operatively coupled to the lens to provide vibration thereto to remove fluids and debris from the lens, and an ultrasonic generator providing driving signals to the ultrasonic transducer.

In an aspect, the cleaning assembly may further include a transparent film in a superposed relation with the lens.

In another aspect, the ultrasonic transducer may be operatively coupled to the transparent film to provide vibration to the transparent film.

In yet another aspect, the transparent film may be formed of a hydrophobic material.

In still yet another aspect, the ultrasonic generator may be disposed in the housing.

In an aspect, the housing may include a button operatively coupled to the ultrasonic generator to selectively activate the ultrasonic generator.

In another aspect, the ultrasonic transducer may be secured to the lens in a hermetically sealed relation.

In yet another aspect, the ultrasonic transducer may have an annular configuration.

In still yet another aspect, the ultrasonic transducer may be mounted about the elongate tubular shaft.

In an aspect, the cleaning assembly may include a plug configured to be coupled to the distal end portion of the elongate tubular shaft such that the lens is enclosed by the plug.

In another aspect, the plug is formed of a transparent and hydrophobic material.

In yet another aspect, the ultrasonic transducer may be secured to the plug.

In an aspect, the ultrasonic transducer is formed from piezoelectric crystals.

In accordance with another aspect of the disclosure, an endoscope includes a housing coupled to a power source, an elongate tubular shaft extending from the housing and including a distal end portion having a lens, and a cleaning assembly. The cleaning assembly includes an ultrasonic transducer operatively coupled to the lens to provide vibration thereto to remove fluids and debris from the lens, an ultrasonic generator providing driving signals to the ultrasonic transducer, and a spray mechanism configured to supply a washer fluid to the lens.

In an aspect, the spray mechanism may include nozzle directing a stream of the washer fluid across a surface of the lens.

In another aspect, the ultrasonic transducer may be hermetically secured to the lens.

In yet another aspect, the spray mechanism may be coupled to a fluid reservoir.

In still yet another aspect, the fluid reservoir may be disposed within the housing.

In still yet another aspect, the housing may include a button operatively coupled to the spray mechanism and the ultrasonic generator to selectively activate the ultrasonic generator while supplying the washer fluid.

In an aspect, the lens may have a coating including a hydrophobic material.

### BRIEF DESCRIPTION OF DRAWINGS

The above and other aspects and features of this disclosure will become more apparent in view of the following detailed description when taken in conjunction with the accompanying drawings wherein like reference numerals identify similar or identical elements.
FIG. 1 is a perspective view of an endoscope in accordance with the disclosure;
FIG. 2 is an enlarged perspective view of the indicated area of detail of FIG. 1;
FIG. 3 is a partial perspective view of an endoscope in accordance with another aspect of the disclosure;
FIG. 4 is a partial perspective view of an endoscope in accordance with yet another aspect of the disclosure;
FIG. 5 a partial perspective view of an endoscope in accordance with still yet another aspect of the disclosure; and
FIG. 6 is a perspective view of the endoscope of FIG. 1, illustrating use with a cannula assembly.

### DETAILED DESCRIPTION

The minimally invasive viewing instrument disclosed herein is described in detail with reference to the drawings, in which like reference numerals designate identical or corresponding elements in each of the several views.

As used herein, the term "distal" refers to the portion that is being described which is farther from a user, while the term "proximal" refers to the portion that is being described which is closer to a user. In addition, the terms parallel and perpendicular are understood to include relative configurations that are substantially parallel and substantially perpendicular up to about + or - 10 degrees from true parallel and true perpendicular. Further, to the extent consistent, any or all of the aspects detailed herein may be used in conjunction with any or all of the other aspects detailed herein.

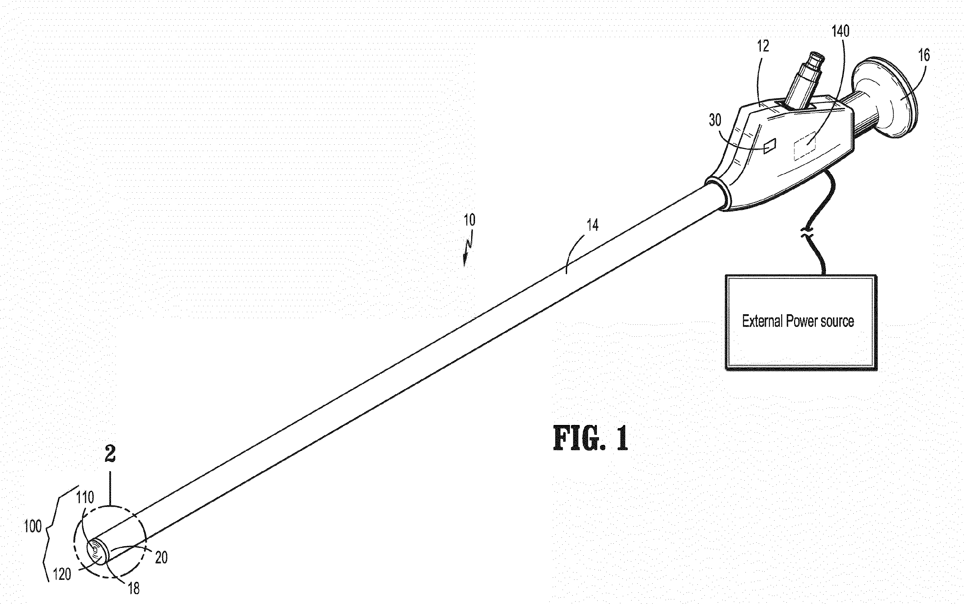

In FIG. 1, an exemplary minimally invasive viewing instrument in accordance with the disclosure is shown generally as an endoscope 10. The endoscope 10 includes a housing 12 and an elongated tubular shaft 14 extending distally from the housing 12 and terminating in a lens 18. The elongate tubular shaft 14 may be rigid, semi-rigid, or flexible. The housing 12 includes a viewfinder 16 adapted to sight images of a surgical field in the patient, e.g. an abdominal cavity, thoracic cavity, etc., as the position of the endoscope 10 is adjusted to view a particular anatomical structure in the surgical field. A camera (not shown) is adapted to receive images of the surgical field sighted through the lens 18 and transmit the images to, e.g., an external monitor, on which the images of the surgical field are displayed. That is, a visual display device converts the optical signal into a video signal to produce a video image on the monitor (or for storage on select media). Accordingly, the monitor enables a clinician to view the anatomical structure in the surgical field inside the patient as the surgical procedure is carried out using minimally invasive or endoscopic surgical instruments. Throughout the surgical procedure, condensation, smoke particles, and biological tissue or matter have a tendency to contact and build up on the lens 18 of the endoscope 10. This tends to obscure the images of the surgical field as they are displayed on the monitor. To this end, the endoscope 10 includes a cleaning assembly 100 that enables cleaning of the lens 18 during the surgical procedure to maintain a clear image without having to remove the endoscope 10 from the patient's body.

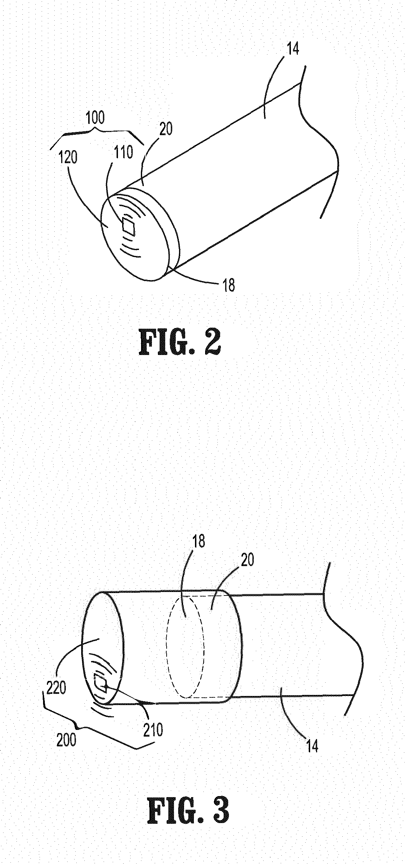

FIG. 2 illustrates the distal tip portion 20 of the elongate tubular shaft 14. The distal tip portion 20 includes a number of optical components (not shown) that produce images of the patient's tissues as known by one skilled in the art. The optical components generally include a window or front element of a lens assembly that is positioned in front of an image sensor (not shown) or in front of a fiber optic imaging guide that transfers an image to the proximal end of the endoscope 10. Illumination sources such as, e.g., light-emitting diodes, fiber optic or illumination guides, may also be provided.

The cleaning assembly 100 includes an ultrasonic transducer 110, a transparent film 120, and an ultrasonic generator 140 (FIG. 1) that provides driving signals to the ultrasonic transducer 110. The ultrasonic transducer 110 is secured to the lens 18 and operatively coupled to the transparent film 120. The transparent film 120 may be formed of a hydrophobic material such as, e.g., a fluoropolymer. The transparent film 120 is disposed in a superposed relation with the lens 18. The ultrasonic transducer 110 is operatively coupled to the ultrasonic generator 140 disposed in the housing 12. The ultrasonic transducer 110 may be formed from one or more piezoelectric crystals that are activated to vibrate upon application of an electronic driving signal from the ultrasound generator 140. For example, a layer of piezoelectric material may be applied to the transparent film 120. Further, the ultrasonic generator 140 is operatively coupled to the button 30 (FIG. 1) that selectively activates the ultrasonic generator 140. In this manner, when the ultrasonic generator 140 is activated, the ultrasonic generator 140 provides driving signals to the ultrasonic transducer 110 such that the ultrasonic transducer 110 provides vibration to the transparent film 120 to remove fluid and/or debris from the transparent film 120 to improve visibility therethrough. The ultrasonic transducer 110 may be one or more ultrasonic transducers 110.

While the cleaning assembly 100 utilizes the transparent film 120, it is also contemplated that the lens 18 may be directly exposed to tissue or the surgical site such that the ultrasonic transducer 110 secured to the lens 18 may vibrate the lens 18 to remove fluid and/or debris from a surface of the lens 18. For example, a layer of piezoelectric material may be applied to the lens 18.

FIG. 3 illustrates a cleaning assembly 200 in accordance with another aspect of the disclosure. Portions of the cleaning assembly 200 that are substantially identical to the portions of the cleaning assembly 100 will not be described. The cleaning assembly 200 includes a plug 220 that is secured to the distal tip portion 20 of the elongate tubular shaft 14. The plug 220 may be detachably secured to the distal tip portion 20 by, e.g., friction fit or snap fit configurations. The plug 220 encloses the lens 18. In this manner, the plug 220 protects the lens 18 from the fluid and/or debris. The plug 220 may be formed of a transparent and hydrophobic material. The plug 220 includes at least one ultrasonic transducer 210. For example, a layer of piezoelectric material may be applied to the plug 220. The ultrasonic transducer 210 is operatively coupled to the ultrasonic generator 140 (FIG. 1) such that when the ultrasonic generator 140 is activated, the ultrasonic generator 140 provides driving signals to the ultrasonic transducer 210 which provides vibration to the plug 220 to remove fluid and/or debris from the plug 220 to improve visibility therethrough.

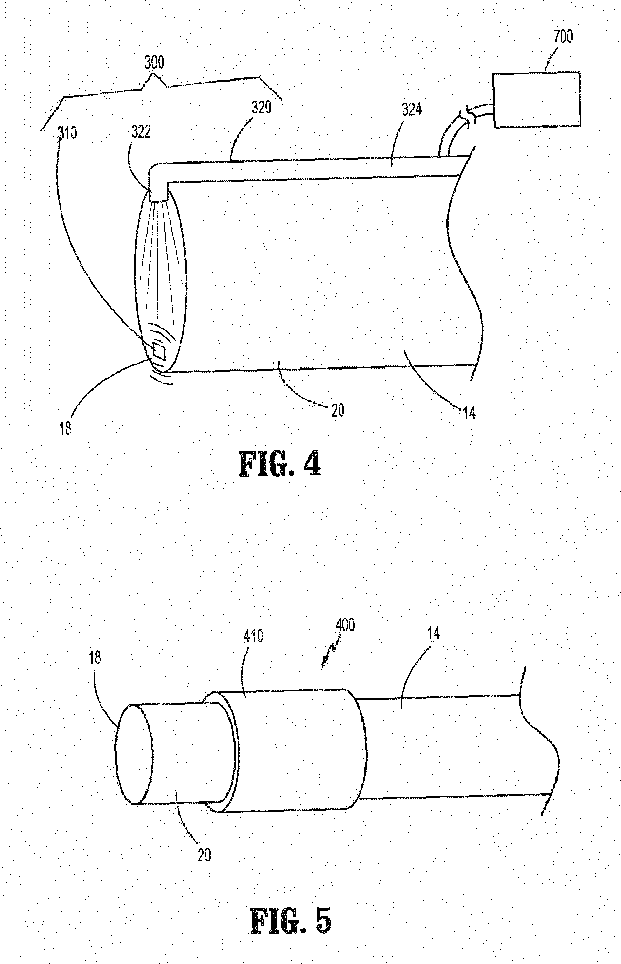

FIG. 4 illustrates a cleaning assembly 300 in accordance with yet another aspect of the disclosure. Portions of the cleaning assembly 300 that are substantially identical to the portions of the cleaning assemblies 100, 200 will not be described. The cleaning assembly 300 includes a transducer 310 secured to the lens 18 and a spray mechanism 320 including a supply line 324 extending along the elongate tubular shaft 14 and coupled to a washer fluid source (not shown), and a nozzle 322 configured to direct a stream of the washer fluid across a surface of the lens 18. For example, a layer of piezoelectric material may be applied to the lens 18. A washer fluid reservoir 700 may be disposed in the housing 12 (FIG. 1) or provided external of the housing 12. The ultrasonic transducer 310 may be in a hermetically sealing relation with the lens 18. Alternatively, the ultrasonic transducer 310 may be disposed within the elongate tubular shaft 14 such that the ultrasonic transducer 310 is protected from the fluids and/or debris. For example, the lens 18 may be coated with a hydrophobic material. The ultrasonic transducer 310 is operatively coupled to the ultrasonic generator 140 (FIG. 1). Under such a configuration when the button 30 (FIG. 1) is pressed by the clinician, the ultrasonic generator 140 is activated, thereby providing driving signals to the ultrasonic transducer 310 which provides vibration to the lens 18 to remove fluid and/or debris from the lens 18 to improve visibility therethrough. Further, the spray mechanism 320 is also operatively coupled to the button 30 such that when the button 30 is pressed by the clinician, the spray mechanism 320 is activated to supply, e.g., a pressurized, washer fluid to the surface of the lens 18. In addition, the supply line 324 may be internal or external of the elongate tubular shaft 14. The spray mechanism 320 may include a pump or a plunger to facilitate supply of the washer fluid.

FIG. 5 illustrates a cleaning assembly 400 in accordance with still yet another aspect of the disclosure. Portions of the cleaning assembly 400 that are substantially identical to the portions of the cleaning assemblies 100, 200, 300 will not be described. The cleaning assembly 400 includes a transducer 410 in a form of an annular sleeve mounted about the elongate tubular shaft 14. Under such a configuration, the lens 18 is directly exposed to the surgical site. The lens 18 may be coated with a hydrophobic material to enhance removal of the fluid and/or debris therefrom. The ultrasonic transducer 410 may be adjustably coupled to the elongate tubular shaft 14 along a length of the elongate tubular shaft 14. The ultrasonic transducer 410 is operatively coupled to the ultrasonic generator 140 (FIG. 1) such that when the ultrasonic generator 140 is activated, the ultrasonic generator 140 provides driving signals to the ultrasonic transducer 410 which provides vibration to the entire elongate tubular shaft 14, which, in turn, removes fluid and/or debris from the lens 18 to improve visibility therethrough.

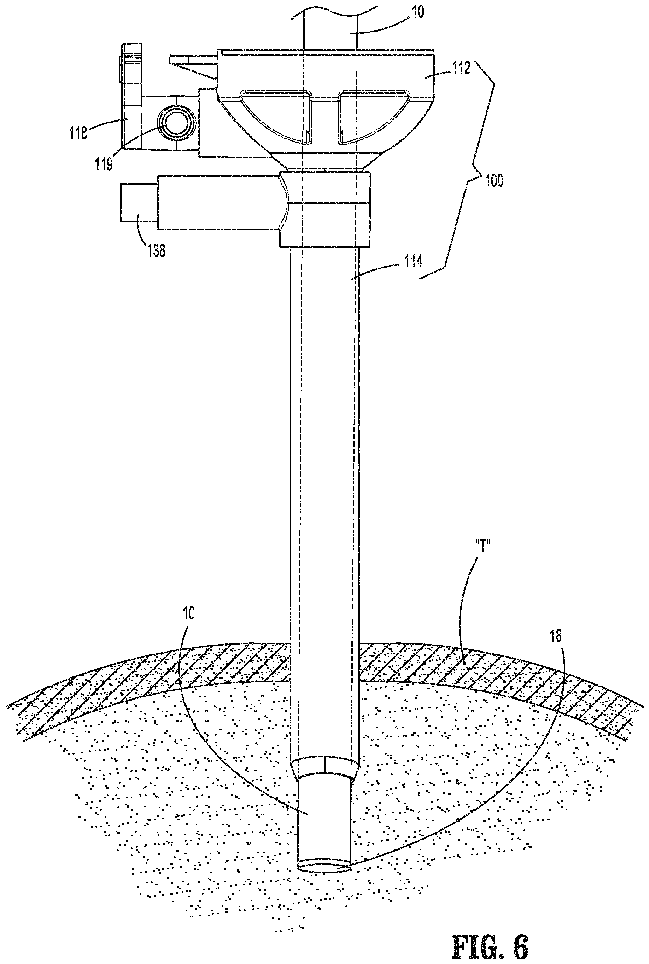

In use, FIG. 6 illustrates the endoscope 10 in use with a cannula assembly 100. The cannula assembly 100 is configured to permit access to, e.g., an insufflated, abdominal cavity during a minimally invasive surgical procedure to permit the introduction of a surgical object for performing various surgical tasks on internal organs within the cavity. The surgical object may be the endoscope 10 or a surgical instrument such as laparoscopic or endoscopic clip appliers, obturators, graspers, dissectors, retractors, staplers, laser probes, photographic devices, tubes, electro-surgical devices and the like. The cannula assembly 100 generally includes a cannula housing 112 and a cannula member 114 extending from the cannula housing 112. The cannula housing 112 is dimensioned for engagement by the clinician and may include one or more internal seals adapted to establish a seal about a surgical object introduced therethrough. The cannula housing 112 also may include an insufflation valve 118 with a fluid connector 119 (e.g., a luer connector) for connecting to a source of insufflation fluids (not shown) for delivery within, e.g., the abdominal cavity. A longitudinal lumen defined by the cannula member 114 is also in fluid communication with the insufflation connector 118 to convey insufflation fluids into the abdominal cavity to establish and/or maintain the pneumoperitoneum. The cannula member 114 further includes a fluid port 138 positioned adjacent the cannula housing 112. The fluid port 138 is adapted to be coupled to a source of inflation fluids to inflate an expandable balloon on the cannula member 114 to seal the expandable balloon against tissue to improve securement of the cannula assembly 100 to tissue and to retain insufflation fluid within the abdominal cavity. The expandable balloon expands radially outwardly upon introduction of inflation fluids through the fluid port 138.

After the cannula assembly 100 is positioned through tissue in a desired orientation, the endoscope 10 may be inserted through the cannula assembly 100 such that the endoscope 10 is directed to the surgical site. When the lens 18 is obscured by the fluids or debris, the clinician may press the button 30 of the housing 12 of the endoscope 10 to clean the lens 18. Specifically, when the clinician presses the button 30, the ultrasonic generator 140 is activated which provides vibration to the lens 18, which, removes the fluids and/or debris on the lens 18. Optionally, pressing of the button 30 (or a plunger) may also dispense the washer fluid onto the lens 18. In this manner, the clinician may improve visibility of the lens 18 without extracting the endoscope 10 from the surgical site, which, in turn, may save operating time and eliminate additional trauma to the patient. The method of use of the cleaning assemblies 200, 300,400 is substantially identical to the method of use of the cleaning system 100, and thus will not be described herein. It is also envisioned that the cleaning assemblies 100, 200, 300, 400 may be adapted for use with a robotic surgical system. For example, the robotic surgical system may activate the transducer or the sprayer mechanism.

It is envisioned that the elongate tubular shaft 14 may include one or more working channels extending through the lens 18 that allow introduction of additional surgical devices into the patient to perform such tasks as obtaining biopsy samples and/or performing surgical procedures.

While the disclosure has been shown in the drawings, it is not intended that the disclosure be limited thereto, as it is intended that the disclosure be as broad in scope as the art will allow and that the specification be read likewise. Therefore, the above description should not be construed as limiting, but merely as exemplifications. Those skilled in the art will envision other modifications within the scope and spirit of the claims appended hereto.

The invention may be described by reference to the following numbered paragraphs:-
1. An endoscope comprising:
   a housing coupled to a power source;
   an elongate tubular shaft extending from the housing and having a distal end portion having a lens; and
   a cleaning assembly including an ultrasonic transducer operatively coupled to the lens to provide vibration thereto to remove fluids and debris from the lens, an ultrasonic generator providing driving signals to the ultrasonic transducer, and a spray mechanism configured to supply a washer fluid to the lens.
2. The endoscope according to paragraph 1, wherein the spray mechanism includes nozzle directing a stream of the washer fluid across a surface of the lens.
3. The endoscope according to paragraph 1, wherein the ultrasonic transducer is hermetically secured to the lens.
4. The endoscope according to paragraph 1, wherein the spray mechanism is coupled to a fluid reservoir.
5. The endoscope according to paragraph 4, wherein the fluid reservoir is disposed within the housing.
6. The endoscope according to paragraph 1, wherein the housing includes a button operatively coupled to the spray mechanism and the ultrasonic generator to selectively activate the ultrasonic generator while supplying the washer fluid.
7. The endoscope according to paragraph 1, wherein the lens has a coating including a hydrophobic material.
8. An endoscope comprising:
   a housing coupled to a power source;
   an elongate tubular shaft extending from the housing and having a distal end portion having a lens; and
   a cleaning assembly including an ultrasonic transducer operatively coupled to the lens to provide vibration thereto to remove fluids and debris from the lens, and an ultrasonic generator providing driving signals to the ultrasonic transducer.
9. The endoscope according to paragraph 8, wherein the cleaning assembly further includes a transparent film in a superposed relation with the lens.
10. The endoscope according to paragraph 9, wherein the ultrasonic transducer is operatively coupled to the transparent film to provide vibration to the transparent film.
11. The endoscope according to paragraph 9, wherein the transparent film is formed of a hydrophobic material.
12. The endoscope according to paragraph 9, wherein the ultrasonic generator is disposed in the housing.
13. The endoscope according to paragraph 9, wherein the housing includes a button operatively coupled to the ultrasonic generator to selectively activate the ultrasonic generator.
14. The endoscope according to paragraph 8, wherein the ultrasonic transducer is secured to the lens in a hermetically sealed relation.
15. The endoscope according to paragraph 8, wherein the ultrasonic transducer has an annular configuration.
16. The endoscope according to paragraph 15, wherein the ultrasonic transducer is mounted about the elongate tubular shaft.
17. The endoscope according to paragraph 8, wherein the cleaning assembly includes a plug configured to be coupled to the distal end portion of the elongate tubular shaft such that the lens is enclosed by the plug.
18. The endoscope according to paragraph 17, wherein the plug is formed of a transparent and hydrophobic material.
19. The endoscope according to paragraph 18, wherein the ultrasonic transducer is secured to the plug.
20. The endoscope according to paragraph 18, wherein the ultrasonic transducer is formed from piezoelectric crystals.

## Claims

1. An endoscope comprising:
a housing coupled to a power source;
an elongate tubular shaft extending from the housing and having a distal end portion having a lens; and
a cleaning assembly including an ultrasonic transducer operatively coupled to the lens to provide vibration thereto to remove fluids and debris from the lens, an ultrasonic generator providing driving signals to the ultrasonic transducer, and a spray mechanism configured to supply a washer fluid to the lens.

2. The endoscope according to claim 1, wherein the spray mechanism includes nozzle directing a stream of the washer fluid across a surface of the lens; and/or wherein the ultrasonic transducer is hermetically secured to the lens; and/or wherein the spray mechanism is coupled to a fluid reservoir.

3. The endoscope according to claim 2, wherein the fluid reservoir is disposed within the housing.

4. The endoscope according to any preceding claim, wherein the housing includes a button operatively coupled to the spray mechanism and the ultrasonic generator to selectively activate the ultrasonic generator while supplying the washer fluid; and/or wherein the lens has a coating including a hydrophobic material.

5. An endoscope comprising:
a housing coupled to a power source;
an elongate tubular shaft extending from the housing and having a distal end portion having a lens; and
a cleaning assembly including an ultrasonic transducer operatively coupled to the lens to provide vibration thereto to remove fluids and debris from the lens, and an ultrasonic generator providing driving signals to the ultrasonic transducer.

6. The endoscope according to claim 5, wherein the cleaning assembly further includes a transparent film in a superposed relation with the lens.

7. The endoscope according to claim 5 or claim 6, wherein the ultrasonic transducer is operatively coupled to the transparent film to provide vibration to the transparent film; and/or wherein the transparent film is formed of a hydrophobic material.

8. The endoscope according to any of claims 5 to 7, wherein the ultrasonic generator is disposed in the housing; preferably wherein the housing includes a button operatively coupled to the ultrasonic generator to selectively activate the ultrasonic generator.

9. The endoscope according to any of claims 5 to 8, wherein the ultrasonic transducer is secured to the lens in a hermetically sealed relation.

10. The endoscope according to any of claims 5 to 9, wherein the ultrasonic transducer has an annular configuration.

11. The endoscope according to any of claims 5 to 10, wherein the ultrasonic transducer is mounted about the elongate tubular shaft.

12. The endoscope according to any of claims 5 to 11, wherein the cleaning assembly includes a plug configured to be coupled to the distal end portion of the elongate tubular shaft such that the lens is enclosed by the plug; preferably wherein the plug is formed of a transparent and hydrophobic material.

13. The endoscope according to claim 12, wherein the ultrasonic transducer is secured to the plug.

14. The endoscope according to claim 13, wherein the ultrasonic transducer is formed from piezoelectric crystals.
